# EUROPÄISCHE PATENTSCHRIFT

(11) **EP 4 158 276 B1**
(45) Veröffentlichungstag und Bekanntmachung des Hinweises auf die Patenterteilung: **28.05.2025**
(21) Anmeldenummer: 21717833.4
(22) Anmeldetag: 08.04.2021
(51) Int. Cl.: G01B 9/02, G01B 9/02056, G01B 9/02091

(54) **ANORDNUNG ZUR DURCHFÜHRUNG EINER OPTISCHEN KOHÄRENZTOMOGRAFIE**
ASSEMBLY FOR CARRYING OUT AN OPTICAL COHERENCE TOMOGRAPHY
ENSEMBLE PERMETTANT LA RÉALISATION D'UNE TOMOGRAPHIE EN COHÉRENCE OPTIQUE

(30) Priorität: 02.06.2020 DE 102020114610
(43) Veröffentlichungstag der Anmeldung: 05.04.2023
(73) Patentinhaber: Heidelberg Engineering GmbH, 69115 Heidelberg (DE)
(72) Erfinder: BRAAF, Boy, 69115 Heidelberg (DE); AUMANN, Silke, 64319 Pfungstadt (DE); MARTENSEN, Björn, 23560 Lübeck (DE); KUTZNER, Lisa, 10315 Berlin (DE); FRITZ, Andreas, 23560 Lübeck (DE)
(74) Vertreter: Wesch, Arno
(86) Internationale Anmeldenummer: PCT/EP2021/059129
(87) Internationale Veröffentlichungsnummer: WO 2021/244794

(56) Entgegenhaltungen:
- US-A1- 2003 174 339
- US-A1- 2004 126 048
- US-A1- 2007 081 166

## Beschreibung

Die Erfindung betrifft eine Anordnung nach dem Oberbegriff des Anspruchs 1.

Aus der US 2007/0081166 A1 ist bereits eine solche Anordnung bekannt. Die US 2004/0126048 A1 zeigt eine Anordnung mit einem polarisationserhaltenden Probenarm und einem polarisationserhaltenden Referenzarm. Die US 2003/0174339 A1 zeigt ein Interferometer, bei dem mindestens ein Arm faseroptisch ausgestaltet ist.

Unter der Bezeichnung optische Kohärenztomografie (in englischer Sprache "Optical Coherence Tomography", üblicherweise abgekürzt durch OCT) wird ein bildgebendes Verfahren verstanden. Mit diesem Verfahren können zwei- und dreidimensionale Bilder aus lichtstreuenden organischen Geweben gewonnen werden.

Bei diesem Verfahren wird üblicherweise Licht mit einer großen Bandbreite und zeitlich geringer Kohärenzlänge in einem Strahlteiler in zwei Teilstrahlen geteilt. Der erste Teilstrahl fällt auf das zu untersuchende organische Gewebe, der zweite Teilstrahl, durchläuft eine Referenzstrecke. Ein dritter Teilstrahl, nämlich Licht, das vom organischen Gewebe reflektiert wird, interferiert mit dem zweiten Teilstrahl in einem Interferometer.

Durch Signale aus der Interferenz lässt sich das Gewebe tiefenaufgelöst, also in der Tiefe der optischen Achse des ersten Teilstrahls, untersuchen. Wenn man das organische Gewebe auch noch flächig bzw. lateral mit dem ersten Teilstrahl abtastet, erhält man dreidimensionale Bilder des organischen Gewebes, sogenannte OCT-Bilder.

Vor diesem Hintergrund ist aus der US 2014/0 176 960 A1 eine Anordnung zur Durchführung einer optischen Kohärenztomografie bekannt geworden, bei der polarisationserhaltende, lichtleitende Fasern ohne Polarisations-Kontrolleinheit zum Einsatz kommen. Insoweit sind Interferometer mit polarisationsstabilen Fasern bereits bekannt.

Ganz allgemein können Interferometer-Anordnungen zur Durchführung einer optischen Kohärenztomografie in zwei unterschiedliche Systemteile aufgeteilt werden. Ein erster Systemteil umfasst einen Kamerakopf, der üblicherweise auf einem Tisch steht und in Richtung des zu untersuchenden Auges ausgerichtet wird.

Ein zweiter Systemteil umfasst zumindest eine Energieversorgungseinheit, die unter dem Tisch und entfernt vom Auge angeordnet ist. Diese Aufteilung wird gewählt, um die Dimensionen und das Gewicht des Kamerakopfs klein zu halten, was insbesondere in Krankenhäusern bevorzugt wird.

Ein flexibles Schlauchkabelpaket verbindet die Energieversorgungseinheit mit dem Kamerakopf. Bei einer Handhabung des Kamerakopfs zur Ausrichtung mit dem zu untersuchenden Auge werden das Schlauchkabelpaket und damit auch lichtleitende Fasern, welche die beiden Systemteile optisch leitend miteinander verbinden, in einer unkontrollierten Weise gebogen.

Übliche lichtleitende Einmodenfasern (in englischer Sprache: "Single-Mode Fibers", abgekürzt durch SMFs) leiten nur transversal zur Ausbreitungsrichtung orientierte Komponenten von Lichtstrahlen.

Sofern zur Verbindung der Systemteile solche Fasern verwendet würden, würde das Biegen des Schlauchkabelpakets unvorhersagbare Polarisationsänderungen in das Interferometer eintragen. Dies kann mit einem erheblichen Verlust des Signal-Rausch-Verhältnisses und damit mit einer verschlechterten Bildqualität eines OCT-Bilds einhergehen.

Der Erfindung liegt daher die Aufgabe zu Grunde, eine Anordnung mit einem Interferometer anzugeben, welche möglichst unanfällig gegen die mit dem Biegen eines Schlauchkabelpakets einhergehenden Effekte ist und ein möglichst gutes Signal-Rausch-Verhältnis oder eine möglichst gute Bildqualität eines OCT-Bilds realisiert.

Die vorliegende Erfindung löst die zuvor genannte Aufgabe durch die Merkmale des Anspruchs 1.

Zunächst ist erkannt worden, dass dem genannten Problem unter anderem dadurch begegnet werden könnte, wenn das gesamte Interferometer, die Lichtquelle, Detektoren und Elektronik eingeschlossen, in einem Gehäuse kombiniert und in den Kamerakopf integriert würden.

Dieses Konzept würde kein flexibles Schlauchkabelpaket mit lichtleitenden oder optischen Fasern zwischen zwei Systemteilen vorsehen. Es würde jedoch zu einem sperrigen und schweren Kamerakopf führen. Darüber hinaus sind eine Miniaturisierung und Integrierung aller Hardwarekomponenten in eine kleindimensionierte Kamera eine große technische Herausforderung.

Weiter wurde erkannt, dass man zwar ein Mittel zur Trennung des Interferometers in zwei Systemteile vorsehen könnte, wobei der Kamerakopf sehr kompakt ist. Das gesamte Interferometer, die Lichtquelle, der bzw. die Detektoren, elektronische Komponenten usw., wären dann in einer Packung außerhalb des Kamerakopfs kombiniert.

Diese Lösung würde eine flexible Kabelverbindung mit optischen Fasern zwischen den zwei Systemteilen erfordern und könnte an Änderungen von Polarisationszuständen und den damit einhergehenden Verlusten des Signal-Rausch-Verhältnisses in einem OCT-Bild leiden.

Es wurde auch erkannt, dass bei dem beschriebenen Trennungsansatz eine aktive Ausrichtung des Polarisationszustands in einem Systemteil durch eine mechanische oder elektrooptische Manipulation der Polarisation notwendig wäre, um Verluste im Signal-Rausch-Verhältnis zu reduzieren.

Die aktive Polarisationsausrichtung ist allerdings üblicherweise ein sehr langsamer Prozess und muss relativ häufig durchgeführt werden.

Die durch die Erfindung vorgeschlagene Lösung erfordert keine häufige Polarisationsausrichtung und leidet daher nicht an zeitraubenden Abläufen, um diese durchzuführen.

Es ist weiter erkannt worden, dass das gesamte Interferometer, die Lichtquelle, der bzw. die Detektoren, elektronische Komponenten usw. eingeschlossen in einer Packung außerhalb des Kamerakopfs integriert werden könnten. Dieser Ansatz würde eine flexible Kabelverbindung mit optischen Fasern zwischen den zwei Systemteilen erfordern und kann, wie beschrieben, an Änderungen von Polarisationszuständen und den damit einhergehenden Verlusten des Signal-Rausch-Verhältnisses in einem OCT-Bild leiden.

Bei diesem Ansatz könnten OCT-Signale in zwei getrennte Komponenten mit unterschiedlicher Polarisation mittels polarisationssensitiven optischen Teilern aufgeteilt und durch zwei getrennte Detektoren gemessen werden. Jegliche Änderungen im Polarisationszustand in einem Interferometer führen zu einer Umverteilung der OCT-Signale über die zwei Detektoren ohne Signalverlust. Dieser Ansatz würde aber zwei optische Detektoren und die mit diesen einhergehende Elektronik erfordern.

Die Messergebnisse würden das Zweifache des Datenvolumens einnehmen, welches bei der erfindungsgemäßen Lösung anfällt. Die Erfindung aber führt zu reduzierten Hardwarekosten und zu einem geringen Datenanfall.

Es wurde auch erkannt, dass ein sogenanntes Common-Path Interferometer verwendet werden könnte, welches das gesamte Interferometer in einem faseroptischen (Koppler) Pfad integriert.

Dieser spezielle Typ eines Interferometers integriert die Arme des Interferometers, nämlich den Probenarm und den Referenzarm, in einen einzigen optischen Pfad und ist daher unanfällig gegen Verluste des Signal-Rausch-Verhältnisses eines OCT-Bildes aufgrund von Änderungen in der Polarisation.

Es wurde aber erkannt, dass das Design des Common-Path Interferometers nur in komplizierter Weise für eine optimale OCT-Detektierung in einem Bulk-Optik basierten Mikroskop oder einer Ophthalmoskop-Schnittstelle zu konstruieren ist, da keine klare physikalische Trennung zwischen den zwei Armen des Interferometers erfolgt.

Für OCT-Mikroskope and Ophthalmoskope ist es aber erforderlich, dass die Länge des Referenzarms des Interferometers verändert werden kann, um an die Tiefenlokalisierung des abgebildeten Objekts bzw. an das Objekt im Probenarm des Interferometers angepasst werden zu können. Dies ist beim Common-Path Interferometer nicht möglich. Zusätzlich erschwert es der geteilte optische Pfad im Common-Path Interferometer, simultan optische Verluste in beiden Armen zu minimieren, was stets in einer bestimmten Form an Verlusten des Signal-Rausch-Verhältnisses in den OCT-Bildern resultieren wird.

Bei der durch die Erfindung vorgeschlagenen Lösung kann bevorzugt eine Konfigurierung nach Art eines Mach-Zehnder-Interferometers oder Michaelson-Interferometers verwendet werden, mit welchen die oben beschriebenen Probleme des Common-Path Interferometers gerade nicht einhergehen.

Auch wurde erkannt, dass nach einem Ansatz ein alternatives faser-basiertes Interferometer verwendet werden könnte, welches polarisationserhaltende lichtleitende Fasern (PMFs) anstelle von Einmodenfasern (SMFs) verwendet. Das Licht, das durch PMFs läuft, wird nicht Änderungen des Polarisationszustands unterworfen, wenn die PMF gebogen oder bewegt wird.

Jedoch machen die physikalischen Eigenschaften eines PMFs, insbesondere der Unterschied zwischen den Brechungsindizes der zwei Kristallachsen dieses optischen Fasertyps, das Interferometer anfällig gegen Geisterartefakte, die ihren Ursprung in Interferenzen zwischen den optischen Signalen haben, die über die zwei Kristallachsen geleitet werden.

Eine Entfernung oder Milderung dieser Geisterartefakte erfordert komplexe Lösungen, die nicht praktikabel sind.

Die durch die Erfindung vorgeschlagene Lösung verwendet dennoch ein faser-basiertes Interferometer, welches überwiegend auf der Verwendung von SMFs in einer überwiegenden Zahl und einem speziell gestalteten Lichtleiter basiert, welches die genannten Geisterartefakte nicht erzeugt. Überraschend können durch die Verbindung zweier polarisationserhaltender lichtleitender Fasern zu einem einzigen Lichtleiter Geisterartefakte vermieden werden.

Die Erfindung realisiert so einen Weg, mit dem ein OCT-Interferometer mittels einer flexiblen optischen Faserverbindung in zwei Interferometerteile aufgespaltet werden kann, die unempfindlich gegen eine Manipulation der Faserverbindung sind.

Erfindungsgemäß ist hierbei erkannt worden, dass die Aufteilung eines Interferometers in zwei Interferometerteile und die Verbindung dieser Interferometerteile unter Erhalt einer Polarisationsstabilität realisiert werden kann.

Es ist erfindungsgemäß eine polarisationsstabile Interferometerkonfigurierung für eine lichtleiter- oder faserbasierte optische Kohärenztomografie realisiert, welche einen kompakten und frei beweglichen Probenarm aufweisen kann.

Erfindungsgemäß wird so ermöglicht, eine Anordnung zu schaffen, bei der ein Interferometerteil als händisch tragbarer, kamerabasierter oder in anderer Weise beweglicher Interferometerteil ausgebildet ist. Dieser Interferometerteil weist geringe und kompakte Dimensionen bei geringem Gewicht auf, da die meisten schwereren Hardwareteile im anderen Interferometerteil platziert werden können, und zwar bevorzugt in einem getrennten abgeschlossenen Gehäuse, welches vom zu untersuchenden Objekt entfernt ist.

Eine erfindungsgemäße Anordnung umfasst konkret ein Interferometer zur Durchführung einer optischen Kohärenztomografie, wobei das Interferometer in zwei voneinander räumlich beabstandete Interferometerteile aufgeteilt ist, wobei die beiden Interferometerteile relativ zueinander bewegbar sind und durch flexible Lichtleiter miteinander optisch verbunden sind, welche den räumlichen Abstand überbrücken. Mindestens einer dieser flexiblen Lichtleiter ist als polarisationserhaltender Lichtleiter ausgestaltet, der aus zwei miteinander verbundenen polarisationserhaltenden lichtleitenden Fasern besteht.

Dieser erste Lichtleiter besteht aus zwei miteinander verbundenen polarisationserhaltenden lichtleitenden Fasern, sogenannten PMFs, deren jeweiligen Kristallachsen um 90° verkippt relativ zueinander angeordnet sind. Die PMFs sind unter Einhaltung eines 90° Versatzes verbunden, so dass eine Kreuzkopplung zwischen den Faserkristallachsen besteht. So wird der erste Lichtleiter, nämlich eine sogenannte xPMF oder Kreuz-PMF, geschaffen.

Dieser erste Lichtleiter kompensiert überraschend das Doppelbrechungsverhalten seiner ersten Komponente mit dem Doppelbrechungsverhalten seiner zweiten Komponente. Daher werden überraschend Geistermuster und feste Störmusterartefakte gemildert, welche durch übliche PMFs bei der Bildgebung in der optischen Kohärenztomografie auftreten. Der erste Lichtleiter erhält einen stabilen Polarisationszustand wie eine übliche PMF aufrecht, kann aber willkürlich gehandhabt bzw. gebogen werden und erzeugt dabei dennoch keine Artefaktprobleme bei der optischen Kohärenztomografie, die übliche PMFs erzeugen würden.

Eine übliche einzelne polarisationserhaltende lichtleitende Faser (abgekürzt PMF oder PM-Faser) ist als Einmodenfaser ausgestaltet, in der linear polarisiertes Licht geleitet wird. Während des Durchtritts des Lichts durch diese Faser bleibt die Polarisation des Lichts erhalten. In solchen lichtleitenden Fasern wird eine erhöhte Doppelbrechung aufrecht erhalten, so dass das Licht in zwei wohldefinierten Polarisationsmoden mit klar unterschiedlichen Phasengeschwindigkeiten entlang der Faser laufen kann. Daher existieren in einer polarisationserhaltenden Faser üblicherweise zwei Hauptachsen oder Kristallachsen, eine langsame und eine schnelle Achse, entlang derer polarisiertes Licht übertragen werden kann, wobei dessen Polarisationszustand erhalten bleibt. Durch das Verkippen dieser zweier Achsen um 90° relativ zu zwei Achsen einer weiteren lichtleitenden Faser werden Geisterartefakte vermieden.

Die beiden polarisationserhaltenden lichtleitenden Fasern könnten durch Spleißen miteinander verbunden sein. Beim Spleißen zweier Fasern werden die Fasern miteinander verschmolzen oder verschweißt. Bevorzugt sind keine weiteren Stoffe zur Herstellung der Verbindung der Fasern notwendig. Aus der EP 0 427 705 A1 ist bekannt, wie das Spleißen zweier Fasern prinzipiell durchgeführt werden kann. Bevorzugt sind die polarisationserhaltenden lichtleitenden Fasern gleich lang ausgebildet. Die Doppelbrechungen der beiden gleich langen Fasern heben sich gegenseitig weg bzw. kompensieren einander. Die beiden polarisationserhaltenden lichtleitenden Fasern sind hintereinander angeordnet und an einem Spleißpunkt miteinander verbunden.

Mindestens ein zweiter und/ oder dritter Lichtleiter ist bzw. sind als Einmodenfaser oder Einmodenfasern ausgestaltet. Der zweite Lichtleiter und/ oder der dritte Lichtleiter können Licht vom bildgebenden Interferometerteil zu einem Detektor im anderen Interferometerteil leiten. Das durch diese Einmodenfasern geleitete Licht muss nicht polarisiert sein bzw. ist bevorzugt nicht polarisiert. Diese Einmodenfasern oder auch abgekürzt SMFs halten im Gegensatz zu den PMFs einen Polarisationszustand des geleiteten Lichts nicht aufrecht.

Vor diesem Hintergrund könnten die Lichtleiter in einem flexiblen Schlauchkabel aufgenommen sein, welches sich zwischen den beiden Interferometerteilen erstreckt. Ein flexibles Schlauchkabel umgibt alle Lichtleiter und eventuell weitere elektrische Leitungen röhrenartig und schützt diese vor einem Abreißen oder anderen Beschädigungen. Das flexible Schlauchkabel besteht aus einem Elastomer oder einem sehr biegsamen Kunststoff, so dass ein Kamerakopf leicht durch Verbiegen und/ oder Verschieben des Schlauchkabels händisch bewegt werden kann.

Der erste Interferometerteil ist einer Energieversorgungseinheit zugeordnet. So kann eine relativ schwere und sperrige Energieversorgungseinheit als stationäres Element auf einer stabilen Unterlage, bevorzugt auf einem Fußboden, angeordnet werden, während andere optische Elemente händisch relativ zum ersten Interferometerteil bewegt werden können.

Vor diesem Hintergrund ist der zweite Interferometerteil einem relativ zum ersten Interferometerteil bewegbaren Kamerakopf zugeordnet. So kann der Kamerakopf problemlos auf ein zu untersuchendes Auge ausgerichtet werden.

Der erste Interferometerteil ist in einem ersten Gehäuse aufgenommen, welches elektronische Komponenten und eine Energieversorgungseinheit umfasst. So können schwere Gegenstände im ersten, stationären Gehäuse aufgenommen werden.

Der zweite Interferometerteil ist in einem zweiten Gehäuse aufgenommen, welches den Kamerakopf umfasst. So kann der Kamerakopf problemlos auf ein zu untersuchendes Auge ausgerichtet werden. Es ist denkbar, dass das zweite Gehäuse den Kamerakopf ausbildet und der zweite Interferometerteil in diesem Gehäuse und damit im Kamerakopf integriert ist.

Der Probenarm und der Referenzarm, längs deren jeweils Licht geführt wird, sind vollständig im zweiten Gehäuse oder im Kamerakopf angeordnet. So wird die Bildgebung nicht durch die Bewegung des Kamerakopfs gestört.

Bevorzugt wird die Anordnung der hier beschriebenen Art in der Augenheilkunde oder der Augenuntersuchung verwendet.

In der Zeichnung zeigen
- Fig. 1: eine schematische Darstellung einer Anordnung mit zwei räumlich voneinander getrennten Interferometerteilen,
- Fig. 2: die Anordnung gemäß Fig. 1, wobei die Interferometerteile und das Schlauchkabelpaket getrennt dargestellt sind, und
- Fig. 3: eine schematische Darstellung des Spleißens zweier gleich langer polarisationserhaltender lichtleitender Fasern, deren Kristallachsen am Spleißpunkt um 90° relativ zueinander verkippt sind.

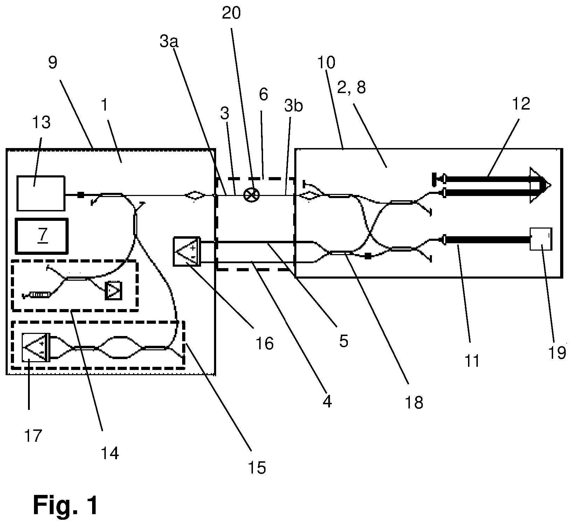

Fig. 1 zeigt eine Anordnung, umfassend ein Interferometer zur Durchführung einer optischen Kohärenztomografie, wobei das Interferometer in zwei voneinander räumlich beabstandete Interferometerteile 1, 2 aufgeteilt ist, wobei die beiden Interferometerteile 1, 2 relativ zueinander bewegbar sind und durch flexible Lichtleiter 3, 4, 5 miteinander optisch verbunden sind, welche den räumlichen Abstand der Interferometerteile 1, 2 überbrücken.

Mindestens ein erster flexibler Lichtleiter 3 ist als polarisationserhaltender Lichtleiter ausgestaltet, der aus zwei miteinander verbundenen gleich langen polarisationserhaltenden lichtleitenden Fasern 3a, 3b, sogenannten PMFs, besteht.

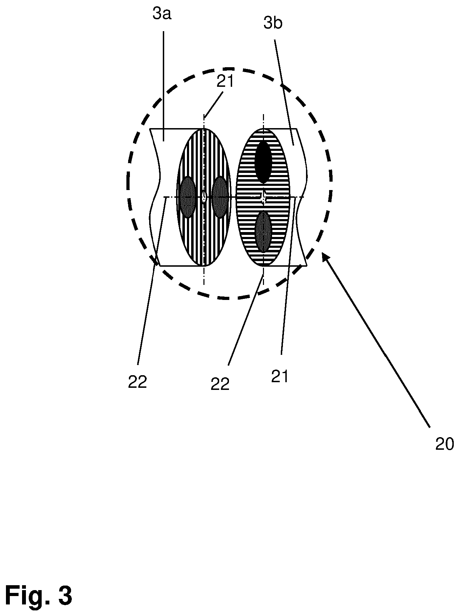

Der erste Lichtleiter 3 besteht aus zwei miteinander verbundenen gleich langen polarisationserhaltenden lichtleitenden Fasern 3a, 3b, deren jeweiligen Kristallachsen 21, 22 an einem Spleißpunkt 20 um 90° verkippt relativ zueinander angeordnet sind. Dies ist in Fig. 3 schematisch dargestellt.

Die beiden gleich langen polarisationserhaltenden lichtleitenden Fasern 3a, 3b, welche den ersten Lichtleiter 3 bilden, sind durch Spleißen am Spleißpunkt 20 miteinander verbunden. Der Spleißpunkt 20 liegt in der Mitte des Lichtleiters 3, der durch die beiden gleich langen hintereinander angeordneten polarisationserhaltenden lichtleitenden Fasern 3a, 3b gebildet ist.

Des Weiteren sind ein zweiter Lichtleiter 4 und ein dritter Lichtleiter 5 als Einmodenfasern ausgestaltet. Alle Lichtleiter 3, 4, 5 sind in einem flexiblen Schlauchkabel 6 aufgenommen, welches sich zwischen den beiden Interferometerteilen 1, 2 erstreckt, und bilden gemeinsam mit dem Schlauchkabel 6 ein flexibel deformierbares Schlauchkabelpaket. Der Spleißpunkt 20 ist in der Mitte oder etwa in der Mitte des Schlauchkabels 6 positioniert.

Der erste Interferometerteil 1 ist einer Energieversorgungseinheit 7 zugeordnet. Der zweite Interferometerteil 2 ist einem bewegbaren Kamerakopf 8 zugeordnet.

Der erste Interferometerteil 1 ist in einem ersten Gehäuse 9 aufgenommen, das elektronische Komponenten und die Energieversorgungseinheit 7 umfasst. Der zweite Interferometerteil 2 ist in einem zweiten Gehäuse 10 aufgenommen, das den Kamerakopf 8 umfasst oder bildet. Der Probenarm 11 und der Referenzarm 12 sind vollständig und nur im zweiten Gehäuse 10 oder im Kamerakopf 8 angeordnet.

Fig 1 zeigt konkret, dass die Anordnung in zwei Interferometerteile 1, 2 aufgeteilt ist, die jeweils im Wesentlichen der Energieversorgungseinheit 7 und dem Kamerakopf 8 zugeordnet sind. Der erste Interferometerteil 1 enthält alle Wesentlichen elektronischen Komponenten, welche die Lichtquelle 13 oder OCT-Lichtquelle, Trigger-Kreise 14 und Clocking-Kreise 15, DAQ-Elektronik und Detektoren 16, 17 umfassen. Der erste Interferometerteil 1 enthält daher alle oder nahezu alle elektronischen Bauteile, die dem ersten Interferometerteil 1 sinnvoll zuordenbar sind.

Konkret enthält der erste Interferometerteil 1 die OCT-Lichtquelle, nämlich eine dem Fachmann als VCSEL Swept-Source ("Vertical Cavity Surface Emitting Laser Swept-Source") bekannte Laserlichtquelle, die Licht mit einer Wellenlänge von 1050 nm abstrahlt, den Trigger-Kreis 14, der als Scan-Trigger-Kreis ausgebildet ist, den Clocking-Kreis 15, nämlich einen sogenannten K-Clock-Kreis, DAQ-Elektronik und einen balancierten Detektor 16 des Interferometers.

Der zweite Interferometerteil 2 ist in den Kamerakopf 8 integriert und enthält im Wesentlichen nur passive Faserkomponenten des gesamten Interferometers.

Drei Lichtleiter 3, 4, 5 verlaufen durch das flexible Schlauchkabel 6 und bilden gemeinsam mit diesem das flexibel deformierbare Schlauchkabelpaket, um die zwei Interferometerteile 1, 2 miteinander optisch zu verbinden. Der erste Lichtleiter 3 leitet Licht von der Lichtquelle 13, nämlich der OCT-Lichtquelle, zum Kamerakopf 8 und ist aus zwei gleich langen polarisationserhaltenden, lichtleitenden Fasern 3a, 3b, nämlich den PMFs, gefertigt.

PMF heißt in englischer Sprache "Polarization-Maintaining Fiber" und wird durch PMF abgekürzt. Der erste Lichtleiter 3 ist durch Spleißen zweier PMFs 3a, 3b gleicher Länge erzeugt.

Der zweite Lichtleiter 4 und der dritte Lichtleiter 5 leiten Licht vom bildgebenden Interferometerteil 2 im Kamerakopf 8 zu dem balancierten Detektor 16 im ersten Interferometerteil 1 bzw. Gehäuse 1, in dem die Energieversorgungseinheit 7 angeordnet ist. Der balancierte Detektor 16 ist für den Polarisationszustand des empfangenen Lichts unempfindlich und PIN Dioden detektieren lediglich dessen Intensität.

Es sind daher übliche Einmodenfasern, welche in englischer Sprache "Single Mode Fibers" heißen und durch SMF abgekürzt werden, zur optischen Verbindung des balancierten Detektors 16 im ersten Interferometerteil 1 mit den zwei Ausgängen des 50/50-Kopplers 18 des zweiten Interferometerteils 2 im Kamerakopf 8 verwendet, obwohl die SMFs unbekannte Änderungen des Polarisationsstatus ausführen, wenn deren Handhabung bzw. Biegung erfolgt.

Es ist daher möglich, den zweiten und dritten Lichtleiter 4, 5 willkürlich zu handhaben, ohne einen Effekt auf die Qualität eines Bildes der optischen Kohärenztomografie befürchten zu müssen. Die Verwendung dieser beiden Lichtleiter 4, 5 in Kombination mit dem ersten Lichtleiter 3, der eine Polarisationsstabilität aufrecht erhält, ist daher besonders vorteilhaft.

Da die drei Lichtleiter 3, 4, 5 entweder unempfindlich gegen Änderungen des Polarisationszustands sind oder die Detektierung ihrer Signale unempfindlich gegen Polarisationsänderungen ist, kann das Schlauchkabelpaket alle drei Lichtleiter 3, 4, 5 enthalten und kann willkürlich bewegt und gehandhabt werden, ohne einen Effekt auf die Signalqualität der optischen Kohärenztomografie befürchten zu müssen.

Die hier beschriebene Interferometerkonfigurierung erfordert eine einmalige Anpassung und ist im weiteren Betrieb stabil. Sie erfordert keine periodische oder Echtzeit-Polarisationsoptimierung. Zusätzlich wird der Raum minimiert, der im Kamerakopf 8 verwendet wird. Hierdurch wird die Konfigurierung sowohl der Energieversorgungseinheit 7 als auch des Kamerakopfs 8 optimiert.

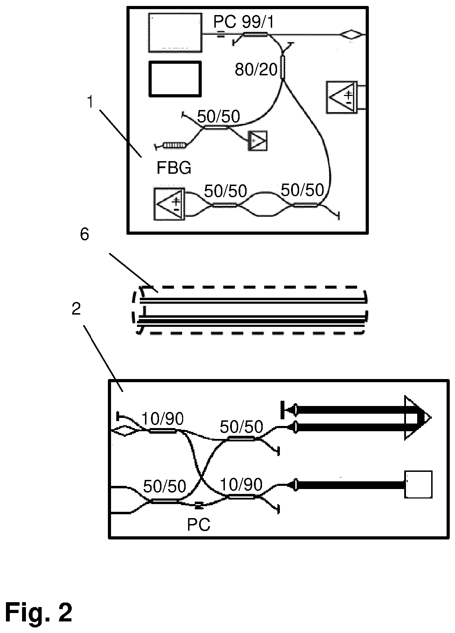

Fig. 2 zeigt schematisch die beiden Interferometerteile 1, 2 und das Schlauchkabelpaket 6 sowie dem Fachmann geläufige übliche elektronische, optische oder optoelektronische Bauelemente eines Interferometers.

Fig. 3 zeigt anhand des Spleißpunkts 20 schematisch, dass es beim Spleißen der zwei gleich langen polarisationserhaltenden lichtleitenden Fasern 3a, 3b notwendig ist, die Orientierungen ihrer Kristallachsen 21, 22 am Spleißpunkt 20 aufeinander abzustimmen. Ein bewusster Versatz von 90°, wie in Fig. 3 dargestellt, wird an dem Spleißpunkt 20 erzeugt, um den hier beschriebenen Effekt der Doppelbrechungskompensierung zu erzielen.

Ein nicht gezeigter PM-Faser-Fusions-Spleißer weist üblicherweise eine Vorrichtung auf, um zu verbindende polarisationserhaltende lichtleitende Fasern geeignet zu rotieren und relativ zueinander auszurichten.

### Bezugszeichenliste:

- 1: Erster Interferometerteil
- 2: Zweiter Interferometerteil
- 3: Erster Lichtleiter
- 3a, 3b: polarisationserhaltende lichtleitende Faser
- 4: Zweiter Lichtleiter
- 5: Dritter Lichtleiter
- 6: Flexibles Schlauchkabel
- 7: Energieversorgungseinheit
- 8: Kamerakopf
- 9: Erstes Gehäuse
- 10: Zweites Gehäuse
- 11: Probenarm
- 12: Referenzarm
- 13: Lichtquelle
- 14: Trigger-Kreis
- 15: Clocking-Kreis
- 16: Balancierter Detektor 16
- 17: Weiterer Detektor
- 18: 50/50-Koppler
- 19: Scan-Einheit
- 20: Spleißpunkt
- 21: Erste Kristallachse
- 22: Zweite Kristallachse

## Patentansprüche

1. Anordnung, umfassend ein Interferometer zur Durchführung einer optischen Kohärenztomografie, wobei das Interferometer in zwei voneinander räumlich beabstandete Interferometerteile (1, 2) aufgeteilt ist, wobei die beiden Interferometerteile (1, 2) relativ zueinander bewegbar sind und durch flexible Lichtleiter (3, 4, 5) miteinander optisch verbunden sind, welche den räumlichen Abstand überbrücken, wobei der erste Interferometerteil (1) in einem ersten Gehäuse (9) aufgenommen ist, welches elektronische Komponenten und eine Energieversorgungseinheit (7) umfasst, wobei der zweite Interferometerteil (2) in einem zweiten Gehäuse (10) aufgenommen ist, welches einen Kamerakopf (8) umfasst, und wobei der Probenarm (11) und der Referenzarm (12) im zweiten Gehäuse (10) oder im Kamerakopf (8) angeordnet sind,
**dadurch gekennzeichnet, dass** mindestens ein erster flexibler Lichtleiter (3) als polarisationserhaltender Lichtleiter ausgestaltet ist, der aus zwei miteinander verbundenen polarisationserhaltenden lichtleitenden Fasern (3a, 3b) besteht, deren jeweiligen Kristallachsen um 90° verkippt relativ zueinander angeordnet sind.

2. Anordnung nach Anspruch 1, **dadurch gekennzeichnet, dass** die beiden polarisationserhaltenden lichtleitenden Fasern (3a, 3b) durch Spleißen miteinander verbunden und/ oder gleich lang ausgebildet sind.

3. Anordnung nach einem der voranstehenden Ansprüche, **dadurch gekennzeichnet, dass** mindestens ein zweiter Lichtleiter (4) und/ oder dritter Lichtleiter (5) als Einmodenfaser bzw. Einmodenfasern ausgestaltet ist bzw. sind.

4. Anordnung nach einem der voranstehenden Ansprüche, **dadurch gekennzeichnet, dass** die Lichtleiter (3, 4, 5) in einem flexiblen Schlauchkabel (6) aufgenommen sind, welches sich zwischen den beiden Interferometerteilen (1, 2) erstreckt.

## Claims

1. An assembly comprising an interferometer for carrying out an optical coherence tomography, wherein the interferometer is divided into two interferometer parts (1, 2) at a spatial distance from each other, wherein the two interferometer parts (1, 2) are movable relative to each other and are optically connected to each other by flexible light guides (3, 4, 5) which bridge the spatial distance, wherein the first interferometer part (1) is accommodated in a first housing (9)comprising electronic components and a power supply unit (7), wherein the second interferometer part (2) is accommodated in a second housing (10) comprising a camera head (8), and wherein the sample arm (11) and the reference arm (12) are arranged in the second housing (10) or in the camera head (8),**characterized in that** at least a first flexible light guide (3) is designed as a polarization-maintaining light guide which consists of two polarization-maintaining light-guiding fibers (3a, 3b) connected to each other, the respective crystal axes of which are arranged tilted by 90° relative to each other.

2. The assembly as claimed in claim 1, **characterized in that** the two polarization-maintaining light-guiding fibers (3a, 3b) are connected to each other by splicing and/or are of equal length.

3. The assembly as claimed in any one of the preceding claims, **characterized in that** at least a second light guide (4) and/or third light guide (5) is or are designed as a single-mode fiber or as single-mode fibers.

4. The assembly as claimed in any one of the preceding claims, **characterized in that** the light guides (3, 4, 5) are accommodated in a flexible hose cable (6) which extends between the two interferometer parts (1, 2).

## Revendications

1. Dispositif comprenant un interféromètre pour réaliser une tomographie par cohérence optique, l'interféromètre étant divisé en deux parties d'interféromètre (1, 2) spatialement distantes l'une de l'autre, les deux parties d'interféromètre (1, 2) étant mobiles l'une par rapport à l'autre et étant reliées optiquement l'une à l'autre par des guides de lumière flexibles (3, 4, 5) qui comblent la distance spatiale, la première partie d'interféromètre (1) étant logée dans un premier boîtier (9) qui comprend des composants électroniques et une unité (7) d'alimentation en énergie, la deuxième partie d'interféromètre (2) étant logée dans un deuxième boîtier (10) qui comprend une tête de caméra (8), et le bras porte-échantillon (11) et le bras de référence (12) étant agencés dans le deuxième boîtier (10) ou dans la tête de caméra (8),
**caractérisé en ce qu'**au moins une première fibre optique flexible (3) est conçue sous la forme d'une fibre optique à maintien de polarisation, qui se compose de deux fibres optiques à maintien de polarisation (3a, 3b) reliées entre elles, dont les axes cristallins respectifs sont agencés de manière inclinée à 90° l'un par rapport à l'autre.

2. Dispositif selon la revendication 1, **caractérisé en ce que** les deux fibres optiques à maintien de polarisation (3a, 3b) sont reliées entre elles par épissure et/ou ont la même longueur.

3. Dispositif selon l'une des revendications précédentes, **caractérisé en ce qu'**au moins une deuxième fibre optique (4) et/ou une troisième fibre optique (5) est/sont configurée(s) sous la forme de fibre monomode ou de fibres monomodes.

4. Dispositif selon l'une des revendications précédentes, **caractérisé en ce que** les fibres optiques (3, 4, 5) sont logées dans un câble flexible (6) qui s'étend entre les deux parties d'interféromètre (1, 2).
